# EUROPÄISCHE PATENTANMELDUNG

(11) **EP 1 632 194 A1**
(43) Veröffentlichungstag der Anmeldung: **08.03.2006**
(21) Anmeldenummer: 04021101.3
(22) Anmeldetag: 06.09.2004
(51) Int. Cl.: A61B 19/00, A61B 5/06, A61B 6/12

(54) **Navigationshilfe und Navigationssystem zur Navigation bei medizinischen Eingriffen**

(71) Anmelder: DKFZ Deutsches Krebsforschungszentrum, 69120 Heidelberg (DE)
(72) Erfinder: Wolf, Ivo, 69257 Wiesenbach (DE); Meinzer, Hans-Peter, Prof.Dr., 69117 Heidelberg (DE); Vetter, Markus, 68123 Edingen (DE)
(74) Vertreter: Isenbruck, Günter

(57) **Zusammenfassung**

Die Erfindung bezieht sich auf eine Navigationshilfe (1) zur Navigation bei medizinischen Eingriffen an nicht-knöchernen Strukturen mit einem nadelförmigen Körper (2), wobei der nadelförmige Körper (2) auf seiner Oberfläche mindestens einen als optischer Marker dienenden länglichen Bereich (4, 5, 6, 13, 14, 15) aufweist, wobei sich der längliche Bereich (4, 5, 6, 13, 14, 15) im Wesentlichen in Längsrichtung des nadelförmigen Körpers (2) erstreckt. Ferner bezieht sich die Erfindung auf ein Verfahren zur Navigation bei medizinischen Eingriffen an nicht-knöchernen Strukturen, wobei mindestens eine erfindungsgemäße Navigationshilfe (1, 9, 10, 11) in die nicht-knöcherne Struktur eingebracht wird, von dem länglichen Bereich (4, 5, 6, 13, 14, 15) reflektiertes oder emittiertes Licht erfasst wird und eine räumliche Lage der mindestens einen Navigationshilfe (1, 9, 10, 11) in 5 Freiheitsgraden anhand des erfassten Lichts ermittelt wird.

## Beschreibung

Die Erfindung bezieht sich auf eine Navigationshilfe, ein Navigationssystem und ein Verfahren zur Navigation bei medizinischen Eingriffen an nicht-knöchernen Strukturen mittels optischer Marker. Sie betrifft die räumliche Lokalisation von nadelförmigen Navigationshilfen und gegebenenfalls von nadelförmigen medizinischen Instrumenten in 5 Freiheitsgraden mittels optischer Verfahren.

Bisher war bei derartigen Eingriffen der Mediziner auf seinen Tast- und Sehsinn beziehungsweise auf die mehrfache Akquisition von intra-interventionell aufgenommenen Bilddaten angewiesen. Dies führte häufig dazu, dass Sicherheitsabstände zu lebenswichtigen Bereichen des Körpers nicht eingehalten werden konnten. Eine besondere Schwierigkeit besteht darin, dass in der Regel Weichteilstrukturen ihre Lage während eines Eingriffs verändern, so dass lebenswichtige Bereiche an einer anderen Position als zu Beginn des Eingriffs zu liegen kommen. Zur Erfassung solcher Änderungen während des Eingriffs hat die mehrfache Akquisition von Bilddaten, zum Beispiel CT- oder Angiographiebilder, den Nachteil einer erhöhten Belastung des Patienten.

Eine genaue Navigation ist jedoch für ein Einhalten von Sicherheitsabständen, insbesondere in der Nähe lebensnotwendiger Strukturen bei diagnostischen und therapeutischen medizinischen Interventionen, zwingend erforderlich.

Die räumliche Lokalisation von nadelförmigen Instrumenten und Navigationshilfen zur Navigation an knöchernen und nicht-knöchernen Strukturen bei diagnostischen und therapeutischen Eingriffen erfolgt im Stand der Technik zum Beispiel mittels optischer Tracking-Systeme, die entweder 3 oder 6 Freiheitsgrade bestimmen. Weitere bereits existierende Tracking-Verfahren, die direkt 5 oder 6 Freiheitsgrade bestimmen können, sind beispielsweise elektromagnetisch, akustisch oder Funk-basierte Tracking-Systeme. Die Verwendung zum Beispiel eines magnetischen Gradientenfeldes hat jedoch den Nachteil, dass während eines medizinischen Eingriffes metallische Gegenstände, beispielweise ein Stahltisch, Haken oder ähnliches, das Tracking-System stören.

Die optische Tracking-Technologie eignet sich bisher nur eingeschränkt, um mehrere nadelförmige Navigationshilfen oder Instrumente in einem beschränkten Zugangsgebiet oder einem beschränkten Diagnose- oder Therapiegebiet mit nicht-knöchernen Strukturen am Patienten zu verwenden. Der Grund hierfür ist der Platzbedarf für die im Stand der Technik verwendeten optischen Fidushal-Marker oder die aktiven Leuchtdioden, die als Referenzpunkte für das optische Tracking-Verfahren dienen. Für die Lokalisation eines medizinischen Instruments oder einer Navigationshilfe in 6 Freiheitsgraden waren hierfür bisher mindestens drei (häufig kugelförmige) optische Marker angeordnet in einer dreidimensionalen Markergeometrie notwendig. Diese optischen Marker können für die Bestimmung von 6 Freiheitsgraden nicht auf einer Geraden liegen. Daher ergab sich eine aufwendige Markergeometrie mit einem hohen Platzbedarf und Gewicht, wodurch diese schwierig zu handhaben waren.

WO 02/34152 bezieht sich auf ein Verfahren zur Navigation bei medizinischen Eingriffen an nicht-knöchernen Strukturen, wobei die nicht-knöcherne Struktur mit ihren präoperativen Daten registriert wird, wenigstens eine Navigationshilfe an oder in der nicht-knöchernen Struktur definiert fixiert wird und anhand der Lage der Navigationshilfe die Lage wenigstens einer Teilstruktur der nicht-knöchernen Struktur ermittelt wird. Die Navigationshilfe enthält dabei Mittel zur Definition eines lokalen Koordinatensystems, die insbesondere Marker umfassen.

Die Aufgabe der vorliegenden Erfindung ist es, die Nachteile des Standes der Technik zu vermeiden und insbesondere das in WO 02/34152 vorgeschlagene Verfahren und die darin vorgeschlagene Navigationshilfe zu verbessern. Die vorliegende Erfindung soll eine objektive und genaue Navigation bei medizinischen Eingriffen an nicht-knöchernen Strukturen mittels optischer Tracking-Technologie ermöglichen.

Diese Aufgabe wird erfindungsgemäß gelöst durch eine Navigationshilfe zur Navigation bei medizinischen Eingriffen an nicht-knöchernen Strukturen mit einem eine Längsachse aufweisenden nadelförmigen Körper , wobei der nadelförmige Körper mindestens einen als optischen Marker dienenden rotationssymmetrischen länglichen Bereich mit einer Symmetrieachse umfasst und die Symmetrieachse des länglichen Bereichs mit der Längsachse des nadelförmigen Körpers zusammenfällt.

Zur Navigation bei medizinischen Eingriffen an nicht-knöchernen Strukturen wird die räumliche Lage der Navigationshilfe in 5 Freiheitsgraden durch eine optische Erfassung der Ausrichtung des länglichen Bereichs ermittelt. Es erfolgt eine automatische beziehungsweise rechnergestützte Lageermittlung und eine dementsprechende Ermittlung der notwendigen Transformation. Hierbei sind 3 Freiheitsgrade notwendig, um den Ort der Navigationshilfe zu bestimmen. Zwei weitere Freiheitsgrade bestimmen die Richtung der Längsachse der Navigationshilfe. Um diese zu bestimmen muss die Orientierung der Navigationshilfe bekannt sein. Mittels der erfindungsgemäßen Navigationshilfe können alle 5 Freiheitsgrade zur räumlichen Navigation von medizinischen Navigationshilfen oder nadelförmigen medizinischen Instrumenten bereitgestellt werden, basierend auf einer optischen Tracking-Technologie. Für die Erfassung der erfindungsgemäßen Navigationshilfe genügt ein in Richtung der Längsachse des nadelförmigen Körpers ausgerichteter länglicher Bereich, der als optischer Marker dient. Unter einem optischen Marker ist dabei ein mittels optischer Verfahren nachweisbares Element zu verstehen, im Falle der vorliegenden Erfindung ein länglicher, optisch erfassbarer Bereich, der Teil des nadelförmigen Körpers der Navigationshilfe ist. Der nadelförmige Körper ist zumindest abschnittsweise im Wesentlichen zylindrisch oder konisch ausgebildet, wobei er zum Beispiel einen kreisförmigen Querschnitt aufweist. Durch den nadelförmigen Körper kann die Navigationshilfe unter minimalen Gewebeverletzungen in eine nicht-knöcherne Struktur eingebracht werden.

Medizinische Eingriffe an nicht-knöchernen Strukturen, bei denen die erfindungsgemäße Navigationshilfe verwendet werden kann, sind zum Beispiel eine Biopsie von Leber, Pankreas oder Mamma oder auch offene oder laparoskopische Operationen an Weichteilorganen.

Die erfindungsgemäße Navigationshilfe hat die Vorteile, dass
- durch die Verwendung des auf die Ausdehnung der Navigationshilfe beziehungsweise ihren nadelförmigen Körper beschränkten, als optischer Marker dienenden länglichen Bereichs die Ausdehnung der erfindungsgemäßen Navigationshilfe gegenüber Navigationshilfen aus dem Stand der Technik mit 3 oder mehr Kugeln oder Leuchtdioden verringert wird,
- eine aufwendige Markergeometrie vermieden wird und dadurch Herstellungskosten und -aufwand gesenkt werden,
- das Gewicht der Navigationshilfe reduziert wird, was zu geringeren Fehlern bei der Bestimmung des Ortes des angrenzenden Weichgewebes bei einem medizinischen Eingriff führt,
- die Messbedingungen verbessert werden, da die gegenüber dem Stand der Technik geringe Anzahl von Markern auch zu einer geringen Anzahl von Phantom-Markern führt. Dadurch wird insbesondere bei mehreren, nahe beieinander in das nicht-knöcherne Gewebe eingebrachten erfindungsgemäßen Navigationshilfen eine deutliche Verbesserung der Messbedingungen erreicht, wodurch sich eine sicherere räumliche Lokalisation der Navigationshilfen ergibt und
- Kollisionen der Markergeometrien besser vermieden werden können. Mechanische Kollisionen führen bei Navigationshilfen aus dem Stand der Technik zu Beschädigungen der Marker oder der zugrundeliegenden Markergeometrie.

In einer bevorzugten Ausführungsform der vorliegenden Erfindung ist der mindestens eine längliche Bereich als eine röhrenförmige Oberflächenschicht oder als Teilabschnitt des nadelförmigen Körpers ausgebildet. Die röhrenförmige Oberflächenschicht umschließt den nadelförmigen Körper entlang eines Teils seiner Länge. Der als Teilabschnitt des nadelförmigen Körpers ausgeführte Bereich ist nicht nur auf der Oberfläche des nadelförmigen Körpers angeordnet, sondern ersetzt Teilabschnitte des röhrenförmigen Körpers vollständig.

Vorzugsweise ist der mindestens eine längliche Bereich Licht emittierend oder reflektierend ausgebildet. Es kann sich bei dem emittierten oder reflektierten Licht um Licht aus dem sichtbaren Bereich oder aus dem infraroten Bereich handeln. Ein reflektierend ausgebildeter Bereich ist ein passiver optischer Marker, ein Licht emittierender Bereich ist ein aktiver optischer Marker. Als aktiver optischer Marker kann bei der vorliegenden Erfindung beispielsweise ein fluoreszierender Bereich dienen.

Vorzugsweise befindet sich derjenige Abschnitt des nadelförmigen Körpers der erfindungsgemäßen Navigationshilfe, der den als optischer Marker dienenden länglichen Bereich aufweist, während des medizinischen Eingriffs nicht im Körperinneren, damit ein optisches Tracking-System das von dem Bereich reflektierte oder aktiv emittierte Licht erfassen kann. Gegebenenfalls kann ein Teil des reflektierenden oder aktiv Licht emittierenden Bereichs während der Intervention durch Gewebe bedeckt sein, wobei dieser bedeckte Teilbereich nicht zur Lokalisation der Navigationshilfe beitragen kann.

Gemäß einer bevorzugten Ausführungsform der vorliegenden Erfindung ist der längliche Bereich, der als optischer Marker dient, derart reflektierend ausgebildet, dass einfallendes Licht in seiner Einfallsrichtung zurückreflektiert wird. Er ist dabei aus einem retroreflektierenden Material ausgebildet, wie es im Stand der Technik bekannt ist.

Gemäß einer bevorzugten Ausführungsform der vorliegenden Erfindung weist der nadelförmige Körper der erfindungsgemäßen Navigationshilfe den länglichen Bereich als Oberflächenschicht in Form einer Beschichtung der Oberfläche oder einer mit der Oberfläche verbundenen Folie auf. Die nadelförmige Navigationshilfe kann zum Beispiel mit einer reflektierenden Schicht, insbesondere mit einer retroreflektierenden Schicht, direkt beschichtet werden. Alternativ dazu kann der Schaft der nadelförmigen Navigationshilfe mit einer reflektierenden Folie überzogen werden. Bei der Folie kann es sich beispielsweise um die Scotchlite® Folie von 3M handeln.

Da bei der vorliegenden Erfindung anstelle von Kugeln, Leuchtdioden oder sonstigen Markern aus dem Stand der Technik ein länglicher Bereich auf der im Wesentlichen zylindrischen oder konischen Ausdehnung der nadelförmigen Navigationshilfe vollständig oder teilweise verwendet wird, gibt der längliche Bereich keine Information über die Orientierung der nadelförmigen Navigationshilfe, insbesondere dann, wenn der längliche Bereich in allen drei Raumrichtungen symmetrisch aufgebaut ist. In diesem Zusammenhang ist unter der Orientierung der Navigationshilfe die binäre Information zu verstehen, welche angibt, in welcher Richtung sich die Spitze der Navigationshilfe orientiert.

Gemäß einer bevorzugten Ausführungsform der vorliegenden Erfindung weist die erfindungsgemäße Navigationshilfe mindestens zwei als optische Marker dienende längliche Bereiche auf, wobei die mindestens zwei Bereiche eine unterschiedliche Länge oder einen unterschiedlichen Durchmesser aufweisen. Durch eine solche Segmentierung des optischen Markers mit dieser unterschiedlichen Gestaltung der mindestens zwei Bereiche, die in Längsrichtung des nadelförmigen Körpers der Navigationshilfe aufeinander folgend angeordnet sind, kann eine eindeutige Information über die Orientierung der Navigationshilfe gewonnen werden.

Ebenso liegt eine eindeutige Information über die Orientierung der Navigationshilfe vor, wenn der längliche Bereich eine konische Form besitzt.

Alternativ dazu kann die Orientierung der nadelförmigen Navigationshilfe mittels Vorwissen über die Orientierungsrichtung bestimmt werden. Dies kann insbesondere im Falle eines einzigen, auf dem nadelförmigen Körper angeordneten durchgehenden länglichen Bereichs genutzt werden. Dabei wird die Orientierung der nadelförmigen Navigationshilfe zum Beispiel durch das Vorwissen über die Patientenlage bestimmt. Der Patient befindet sich bei dem medizinischen Eingriff vorzugsweise auf einem Tisch liegend und es liegt das Vorwissen vor, dass die nadelförmige erfindungsgemäße Navigationshilfe von oben mit nach unten gerichteter Spitze und nicht von unten mit nach oben gerichteter Spitze in die nicht-knöcherne Struktur eingebracht wird, so dass dieses Vorwissen die Information über die Ausrichtung der Spitze der Navigationshilfe enthält. Nach dem Einbringen der Navigationshilfe in die nicht-knöcherne Struktur eines Patienten kann die Orientierung der Navigationshilfe zum Beispiel aufgrund der Bewegung der nicht-knöchernen Struktur durch eine Kontinuitätsannahme auch in eine von unten orientierte Position übergehen.

Die Erfindung bezieht sich weiterhin auf ein Navigationssystem zur Navigation bei medizinischen Eingriffen an nicht-knöchernen Strukturen, wobei das Navigationssystem mindestens zwei erfindungsgemäße Navigationshilfen enthält. Zur Navigation bei medizinischen Eingriffen an nicht-knöchernen Strukturen werden mindestens zwei, vorzugsweise mindestens drei erfindungsgemäße Navigationshilfen eingesetzt. Die mindestens zwei erfindungsgemäßen Navigationshilfen bilden ein eindeutiges Bezugssystem, das eine genaue Navigation während des medizinischen Eingriffs, insbesondere zum Einhalten von Sicherheitsabständen in der Nähe lebensnotwendiger Strukturen, ermöglicht.

Vorzugsweise lassen sich die mindestens zwei erfindungsgemäßen Navigationshilfen über unterschiedliche längliche Bereiche unterscheiden, die insbesondere eine verschiedene Länge, Segmentierung oder einen unterschiedlichen Durchmesser aufweisen können. Die mindestens zwei Navigationshilfen werden in einen begrenzten räumlichen Bereich der nicht-knöchernen Struktur eingebracht und mittels der optischen Tracking-Technologie lokalisiert. Durch die optische Lokalisation können 5 Freiheitsgrade der erfindungsgemäßen Navigationshilfen bestimmt werden.

Das erfindungsgemäße Navigationssystem umfasst vorzugsweise ein medizinisches Instrument mit einem eine Längsachse aufweisenden nadelförmigen Körper, wobei der nadelförmige Körper des medizinischen Instruments auf seiner Oberfläche mindestens einen als optischer Marker dienenden rotationssymmetrischen länglichen Bereich mit einer Symmetrieachse umfasst und die Symmetrieachse des länglichen Bereichs mit der Längsachse des nadelförmigen Körpers zusammenfällt. Auf das medizinische Instrument sind alle oben für die erfindungsgemäße Navigationshilfe getroffenen Aussagen ebenfalls zutreffend, soweit sie anwendbar sind. Das medizinische Instrument ist beispielsweise ein Jet-Cutter, der über einen Wasserstrahl schneiden kann, oder eine RF-Sonde eines Radiofrequenz-Ablators. Das erfindungsgemäße Navigationssystem unter Verwendung von mindestens zwei erfindungsgemäßen Navigationshilfen ermöglicht eine Definition eines über den Operationsverlauf invarianten, lokal exakten Koordinatensystems, das eine Visualisierung des medizinischen Instruments des Operateurs in Relation zu wichtigen Strukturen im zu operierenden Organ ermöglicht.

Das erfindungsgemäße Navigationssystem enthält ferner vorzugsweise Mittel zur Erfassung der jeweiligen räumlichen Lage der Navigationshilfen in 5 Freiheitsgraden. Als Mittel zur Erfassung der jeweiligen räumlichen Lage der Navigationshilfen können beispielsweise IR-Kameras, CCD-Kameras oder sonstige zur Erfassung des von dem länglichen Bereich emittierten oder reflektierten Lichts geeignete Kameras eingesetzt werden. Vorzugsweise enthält das Navigationssystem mindestens zwei Mittel zur Erfassung der jeweiligen räumlichen Lage der Navigationshilfen. Aus den sich ergebenden Linien in den Bildern der mindestens zwei Mittel (z.B. Kamerabildern) und den Kalibrationstransformationen der verschiedenen Mittel (z.B. Kalibrationstransformationen der verschiedenen Kameras) wird die gesuchte Transformation (5 Freiheitsgrade) vorzugsweise in einer Datenverarbeitungsanlage ermittelt.

Das nadelförmige erfindungsgemäße medizinische Instrument oder die nadelförmigen erfindungsgemäßen Navigationshilfen können auch mit herkömmlichen optischen Tracking-Verfahren kombiniert werden. Eine solche Kombination ist beispielsweise sinnvoll, um mittels der erfindungsgemäßen Navigationshilfen die Lage und Verformung eines Weichteilorgans zu erfassen. Mit dem herkömmlichen optischen Tracking-Verfahren kann dabei die Position eines medizinischen Instruments, das beispielsweise nicht nadelförmig aufgebaut ist und folglich 6-dimensional erfasst werden muss, in räumlicher Relation zu einem Organ beziehungsweise zu einer Läsion ermittelt werden.

Die Erfindung bezieht sich weiterhin auf ein Verfahren zur Navigation bei medizinischen Eingriffen an nicht-knöchernen Strukturen, wobei mindestens eine erfindungsgemäße Navigationshilfe in die nicht-knöcherne Struktur eingebracht wird, von dem länglichen Bereich reflektiertes oder emittiertes Licht erfasst wird und eine räumliche Lage der mindestens einen Navigationshilfe in 5 Freiheitsgraden anhand des erfassten Lichts ermittelt wird. Bei dem erfindungsgemäßen Verfahren wird die in die nicht-knöcherne Struktur eingebrachte Navigationshilfe vorzugsweise mit Hilfe eines Weichteilankers fixiert, wobei der Weichteilanker eine translationsinvariente Fixierung der Navigationshilfe (ausreichend bei einer Navigationseinheit mit 5 Freiheitsgraden) in der nicht-knöchernen Struktur ermöglicht. Bei dem Weichteilanker kann es sich zum Beispiel um Hakenklemmen handeln, die aus der Spitze der nadelförmigen Navigationshilfe herausführbar sind.

Das bei dem erfindungsgemäßen Verfahren erfasste Licht wird von dem sich im Wesentlichen in Längsrichtung des nadelförmigen Körpers erstreckenden länglichen Bereich reflektiert oder emittiert. Anhand des erfassten Lichts werden die 5 Freiheitsgrade Ort und Ausrichtung der Navigationshilfe bestimmt. Dabei wird die Orientierung der Navigationshilfe durch entsprechender Gestaltung des mindestens einen länglichen Bereichs oder durch Vorwissen bestimmt. Die Orientierung wird vorzugsweise anhand von durch mindestens zwei längliche Bereiche der Navigationshilfe reflektiertem oder emittiertem Licht ermittelt, wobei die mindestens zwei länglichen Bereiche unterschiedliche Längen oder Durchmesser aufweisen.

Bei einer bevorzugten Ausführungsform des erfindungsgemäßen Verfahrens werden mindestens zwei erfindungsgemäße Navigationshilfen in die nicht-knöcherne Struktur eingebracht, wobei eine Unterscheidung der mindestens zwei Navigationshilfen dadurch erfolgt, dass die mindestens zwei Navigationshilfen längliche Bereiche mit unterschiedlichen Längen oder Durchmessern aufweisen. Eine unterschiedliche Segmentierung der länglichen Bereiche ist ebenfalls denkbar.

Anhand der Figuren wird die Erfindung nachstehend näher erläutert.

Es zeigt
- Figur 1: eine erfindungsgemäße nadelförmige Navigationshilfe oder ein erfindungsgemäßes nadelförmiges medizinisches Instrument mit einem als optischer Marker dienenden länglichen Bereich,
- Figur 2: eine erfindungsgemäße nadelförmige Navigationshilfe oder ein erfindungsgemäßes nadelförmiges medizinisches Instrument mit zwei als optische Marker dienenden länglichen Bereichen und
- Figur 3: ein Organ, in das zur Navigation bei einem medizinischen Eingriff drei erfindungsgemäße Navigationshilfen eingesetzt sind zur Navigation eines erfindungsgemäßen nadelförmigen medizinischen Instruments.

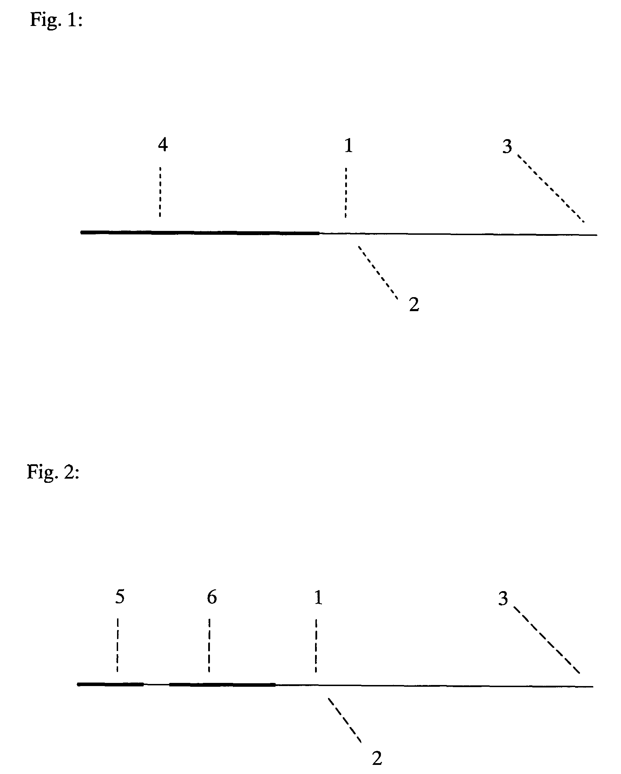

Figur 1 zeigt schematisch eine nadelförmige Navigationshilfe beziehungsweise ein nadelförmiges medizinisches Instrument 1, die beide nachfolgend kurz als Nadel 1 bezeichnet werden. Die Nadel 1 weist einen nadelförmigen Körper 2 mit einer Länge zwischen 5 und 25 cm auf. An einem Ende der Nadel 1 ist eine Spitze 3 angeordnet. An dem der Spitze 3 gegenüberliegenden Ende der Nadel 1 besitzt der nadelförmige Körper 2 auf seiner Oberfläche einen als optischen Marker dienenden länglichen Bereich 4, der beispielsweise als Oberflächenschicht oder als Abschnitt des nadelförmigen Körpers ausgebildet ist. Der längliche Bereich 4 kann als Oberflächenschicht beispielsweise durch eine Beschichtung der Oberfläche des nadelförmigen Körpers 2 oder durch Auftragen einer Folie auf die Oberfläche hergestellt sein. Der längliche Bereich 4 erstreckt sich auf der Oberfläche des nadelförmigen Körpers 2 in Längsrichtung über eine Länge zwischen 2 und 22 cm. Seine Symmetrieachse stimmt mit der Längsachse des nadelförmigen Körpers überein.

Figur 2 zeigt eine weitere Nadel 1 mit einem nadelförmigen Körper 2 und einer Spitze 3, die einen ersten länglichen Bereich 5 und einen zweiten länglichen Bereich 6 aufweist. Diese Segmentierung in einen ersten länglichen Bereich 5 und einen zweiten länglichen Bereich 6 kann einerseits dazu dienen, die Nadel 1 unter einer Vielzahl von Nadeln zu identifizieren, die eine unterschiedliche Segmentierung aufweisen. Des Weiteren kann diese Segmentierung in den ersten länglichen Bereich 5 und den zweiten länglichen Bereich 6 zur Bestimmung der Orientierung der Nadel 1 dienen, da der erste längliche Bereich 5 kürzer als der zweite längliche Bereich 6 ist, sich der jeweilige längliche Bereich 5, 6 daher eindeutig identifizieren lässt und die Lage des ersten kurzen länglichen Bereichs auf der der Spitze 3 entgegengesetzten Seite der Nadel 1 und des zweiten längeren Bereichs auf der der Spitze 3 zugewandten Seite der Nadel 1 bekannt ist.

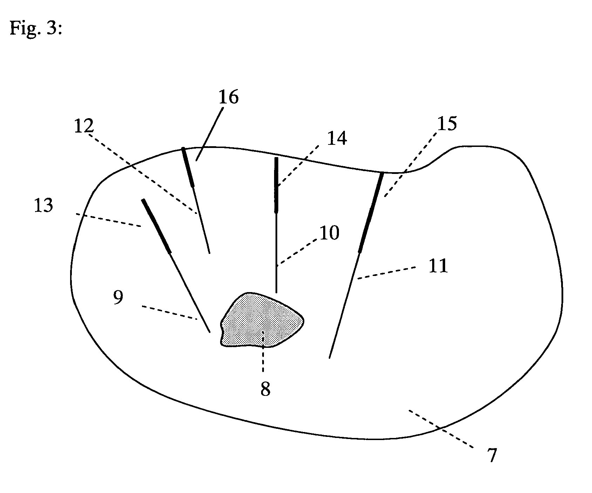

In Figur 3 ist ein Organ 7 dargestellt, das eine nicht-knöcherne Struktur darstellt, und an dem ein medizinischer Eingriff aufgrund eines Tumors 8 erfolgt. Zur Navigation bei diesem medizinischen Eingriff sind in das Organ 7 drei erfindungsgemäße Navigationshilfen 9, 10, 11 eingebracht. Ferner ist in Figur 3 ein bei dem medizinischen Eingriff verwendetes nadelförmiges medizinisches Instrument 12 dargestellt. Die drei Navigationshilfen 9, 10, 11 umfassen jeweils einen nadelförmigen Körper 2, der auf seiner Oberfläche jeweils einen als optischer Marker dienenden länglichen Bereich 13, 14, 15 aufweist. Die drei Navigationshilfen 9, 10, 11 sind rund um den Tumor 8 verteilt, um eine genaue Navigation des medizinischen Instruments 12 zu ermöglichen. Vor der Durchführung des erfindungsgemäßen Verfahrens zur Navigation bei dem medizinischen Eingriff wird mittels eines im Stand der Technik bekannten bildgebenden Verfahrens die Lage des Tumors 8 ermittelt, beispielsweise mittels Ultraschall, CT, MR oder ähnlichem. Während der Durchführung des medizinischen Eingriffs kann die Position des medizinischen Instruments 12 relativ zu dem Tumor 8 zum Beispiel 60 mal pro Sekunde nach dem erfindungsgemäßen Verfahren bestimmt werden.

Zur Unterscheidung der drei Navigationshilfen 9, 10, 11 sind die darauf angeordneten länglichen Bereiche 13, 14, 15 von verschiedener Länge. Das nadelförmige medizinische Instrument 12 weist ebenfalls einen länglichen Bereich 16 als optischen Marker auf. Es kann somit ebenso wie die drei Navigationshilfen 9, 10, 11 mit Hilfe desselben optischen Verfahrens lokalisiert werden.

### Bezugszeichenliste

- 1: Navigationshilfe oder medizinisches Instrument
- 2: nadelförmiger Körper
- 3: Spitze
- 4: länglicher Bereich
- 5: erster länglicher Bereich
- 6: zweiter länglicher Bereich
- 7: Organ
- 8: Tumor
- 9: erste Navigationshilfe
- 10: zweite Navigationshilfe
- 11: dritte Navigationshilfe
- 12: medizinisches Instrument
- 13: länglicher Bereich der ersten Navigationshilfe
- 14: länglicher Bereich der zweiten Navigationshilfe
- 15: länglicher Bereich der dritten Navigationshilfe
- 16: länglicher Bereich des medizinischen Instruments

## Patentansprüche

1. Navigationshilfe zur Navigation bei medizinischen Eingriffen an nicht-knöchernen Strukturen mit einem eine Längsachse aufweisenden nadelförmigen Körper (2), **dadurch gekennzeichnet, dass** der nadelförmige Körper (2) mindestens einen als optischen Marker dienenden rotationssymmetrischen länglichen Bereich (4, 5, 6, 13, 14, 15) mit einer Symmetrieachse umfasst und die Symmetrieachse des länglichen Bereichs (4, 5, 6, 13, 14, 15) mit der Längsachse des nadelförmigen Körpers (2) zusammenfällt.

2. Navigationshilfe gemäß Anspruch 1, **dadurch gekennzeichnet, dass** der mindestens eine längliche Bereich (4, 5, 6, 13, 14, 15) als eine röhrenförmige Oberflächenschicht oder als Teilabschnitt des nadelförmigen Körpers (2) ausgebildet ist.

3. Navigationshilfe gemäß einem der Ansprüche 1 oder 2, **dadurch gekennzeichnet, dass** der mindestens eine längliche Bereich (4, 5, 6, 13, 14, 15) Licht emittierend oder reflektierend ausgebildet ist.

4. Navigationshilfe gemäß Anspruch 3, **dadurch gekennzeichnet, dass** der längliche Bereich (4, 5, 6, 13, 14, 15) derart reflektierend ausgebildet ist, dass einfallendes Licht in seiner Einfallsrichtung zurückreflektiert wird.

5. Navigationshilfe gemäß einem der Ansprüche 1 bis 4, **dadurch gekennzeichnet, dass** der nadelförmige Körper (2) den länglichen Bereich (4, 5, 6, 13, 14, 15) als Schicht in Form einer Beschichtung der Oberfläche oder einer mit der Oberfläche verbundenen Folie aufweist.

6. Navigationshilfe gemäß einem der Ansprüche 1 bis 5, **gekennzeichnet durch** mindestens zwei als optische Marker dienende längliche Bereiche (5, 6), wobei die mindestens zwei länglichen Bereiche (5, 6) eine unterschiedliche Länge oder einen unterschiedlichen Durchmesser aufweisen.

7. Navigationssystem zur Navigation bei medizinischen Eingriffen an nicht-knöchernen Strukturen, **gekennzeichnet durch** mindestens zwei Navigationshilfen (9, 10, 11) gemäß einem der Ansprüche 1 bis 6.

8. Navigationssystem gemäß Anspruch 7, **gekennzeichnet durch** ein medizinisches Instrument (12) mit einem eine Längsachse aufweisenden nadelförmigen Körper (2), wobei der nadelförmige Körper (2) des medizinischen Instruments (12) auf seiner Oberfläche mindestens einen als optischer Marker dienenden rotationssymmetrischen länglichen Bereich (16) mit einer Symmetrieachse umfasst und die Symmetrieachse des länglichen Bereichs (4, 5, 6, 13, 14, 15) mit der Längsachse des nadelförmigen Körpers (2) zusammenfällt.

9. Navigationssystem gemäß einem der Ansprüche 7 oder 8, **gekennzeichnet durch** Mittel zur Erfassung der jeweiligen räumlichen Lage der Navigationshilfen (1, 9, 10, 11) in 5 Freiheitsgraden.

10. Verfahren zur Navigation bei medizinischen Eingriffen an nicht-knöchernen Strukturen, **dadurch gekennzeichnet, dass** mindestens eine Navigationshilfe (1, 9, 10, 11) gemäß einem der Ansprüche 1 bis 6 in die nicht-knöcherne Struktur eingebracht wird, von dem länglichen Bereich (4, 5, 6, 13, 14, 15) reflektiertes oder emittiertes Licht erfasst wird und eine räumliche Lage der mindestens einen Navigationshilfe (1, 9, 10, 11) in 5 Freiheitsgraden anhand des erfassten Lichts ermittelt wird.

11. Verfahren gemäß Anspruch 10, **dadurch gekennzeichnet, dass** die Navigationshilfe (1, 9, 10, 11) eine Orientierung besitzt, die anhand von durch mindestens zwei längliche Bereiche (5, 6) der Navigationshilfe (1) reflektiertem oder emittiertem Licht ermittelt wird, wobei die mindestens zwei länglichen Bereiche (5, 6) unterschiedliche Längen oder Durchmesser aufweisen.

12. Verfahren gemäß Anspruch 10, **dadurch gekennzeichnet, dass** die Navigationshilfe (1, 9, 10, 11) eine Orientierung besitzt, die anhand von Vorwissen ermittelt wird.

13. Verfahren gemäß einem der Ansprüche 10 bis 12, **dadurch gekennzeichnet, dass** mindestens zwei Navigationshilfen (9, 10, 11) in die nicht-knöcherne Struktur eingebracht werden und eine Unterscheidung der mindestens zwei Navigationshilfen (9, 10, 11) **dadurch** erfolgt, dass die mindestens zwei Navigationshilfen (9, 10, 11) längliche Bereiche (13, 14, 15) mit unterschiedlichen Längen, Durchmessern oder Segmentierungen aufweisen.
